# EUROPÄISCHE PATENTANMELDUNG

(11) **EP 4 321 238 A1**
(43) Veröffentlichungstag der Anmeldung: **14.02.2024**
(21) Anmeldenummer: 22189288.8
(22) Anmeldetag: 08.08.2022
(51) Int. Cl.: B01D 53/86, A62D 3/34, A62D 3/37, C07C 19/00

(54) **DEHALOGENIERUNG VON HALOGENIERTEN KOHLENWASSERSTOFFEN**

(71) Anmelder: Grillo-Werke Aktiengesellschaft, 47169 Duisburg (DE)
(72) Erfinder: Ott, Timo, 47169 Duisburg (DE)
(74) Vertreter: dompatent von Kreisler Selting Werner - Partnerschaft von Patent- und Rechtsanwälten mbB

(57) **Zusammenfassung**

Die vorliegende Erfindung betrifft ein Verfahren zur Dehalogenierung von Halogenkohlenstoff-Verbindungen, das dadurch gekennzeichnet ist, dass man die Halogenkohlenstoff-Verbindung in Gegenwart eines Katalysators mit elementarem Wasserstoff in Kontakt bringt, wobei der Katalysator ein Metallkatalysator ist.

## Beschreibung

Die vorliegende Erfindung betrifft ein Verfahren zur Dehalogenierung von halogenierten Kohlenwasserstoffverbindungen.

Halogenkohlenwasserstoffe finden breite Anwendung als Treib- und Kältemittel, als Lösungsmittel und in vielfältigen chemischen Prozessen. Entsprechend fallen je nach Randbedingungen flüssige und gasförmige Abfallströme an, die entsorgt werden müssen.

So fallen beispielsweise Trifluormethan (Fluoroform, CHF₃), Difluormethan (CH₂F₂) und Monofluormethan (FCH₃) als gasförmige Nebenprodukte und Abfallströme zum Beispiel bei der Produktion von PTFE ("Teflon") an. Als extrem starke Klimagase (10.000 bis 20.0000 CO₂-Äquivalente) müssen diese entsprechend verwertet beziehungsweise entsorgt werden.

Die Gruppe von Halogenkohlenwasserstoffen allgemein ist bedenklich im Hinblick auf Arbeits- und Gesundheitsschutz (giftig, cancerogen u.a.) und Umweltschutz (Klima, Ozon, Persistenz). Entsprechend ist eine fachgerechte Entsorgung absolut notwendig.

Die derzeitigen Entsorgungsmethoden spalten die Halogenkohlenwasserstoffe, wie zum Beispiel die Fluorkohlenwasserstoffe (FKW), aus den Abfallstrom thermisch in Verbrennungsöfen oder in einer Plasmakammer. Diese Prozesse finden bei hohen Temperaturen von deutlich über 1000 °C statt. Auf Grund der hohen Temperaturen sind besondere Werkstoffe notwendig, weshalb die Verfahren teuer sind. Auch auf Grund der Produkte, so entsteht beispielsweise CO₂, und der geringen Energieeffizienz der Verfahren, ist die aktuelle Entsorgung als klimaschädlich anzusehen. Zudem können toxische Zwischen- und Nebenprodukte, wie zum Beispiel Phosgen oder Dioxine entstehen, so dass eine aufwändige Überwachung erforderlich ist. Bei der Entsorgung durch thermische Spaltung von FKW entstehen als Produkte wässrige Flusssäure (HF) und CO₂, welche beide keine werthaltigen Produkte darstellen.

Es besteht daher Bedarf an einem Verfahren zur Entsorgung von halogenierten Kohlenwasserstoffen, in welchem diese in weniger klimaschädliche und weniger toxische Verbindungen überführt werden. Gleichzeitig soll das Verfahren nachhaltig sein, und zwar im Hinblick auf die benötigte Energie und Ressourcen sowie nach Möglichkeit auch im Hinblick auf die gewonnen Produkte.

Überraschenderweise hat sich gezeigt, dass eine Dehalogenierung mittels heterogener Katalyse an einem Metallkatalysator möglich ist. In einer ersten Ausführungsform wird die der vorliegenden Erfindung zu Grunde liegende Aufgabe daher gelöst durch ein Verfahren zur Dehalogenierung von Halogenkohlenstoff-Verbindungen, das dadurch gekennzeichnet ist, dass man die Halogenkohlenstoff-Verbindung in Gegenwart eines Katalysators mit elementarem Wasserstoff in Kontakt bringt, wobei der Katalysator ein Metallkatalysator ist.

Das vorliegende Verfahren ermöglicht somit die Entfernung von Halogenen aus organischen Verbindungen. Dabei wird die Halogen-Kohlenstoff-Verbindung getrennt und die so entstehende freie Valenz am Kohlenstoff durch Wasserstoff gesättigt. Es erfolgt somit eine Substitution des Halogenatoms bzw. der Halogenatome in der Verbindung durch Wasserstoff. Dies wird erfindungsgemäß als Dehalogenierung bezeichnet.

Halogen-Kohlenstoff-Verbindungen im Sinne der vorliegenden Erfindung sind insbesondere Kohlenstoff-Verbindungen mit bis zu 12 Kohlenstoffatomen, vorzugsweise handelt es sich um C₁₋₁₀-Verbindungen, bevorzugt um C₁₋₈-Verbindungen. Diese können linear oder verzweigt, gesättigt oder ungesättigt sein. Es handelt sich somit um Halogenalkane, Halogenalkene oder Halogenalkine mit 1 bis 12, vorzugsweise mit 1 bis 10, besonders bevorzugt mit 1 bis 8 Kohlenstoffatomen. Insbesondere sind die Verbindungen gesättigt und können somit als Halogenalkane bezeichnet werden.

Ein oder mehrere Kohlenstoffatome innerhalb der C-Kette können erfindungsgemäß auch durch ein oder mehrere Atome, ausgewählt aus O, N, S, P substituiert sein.

Dabei weist die Verbindung zumindest eine C-Halogen-Bindung auf, welche erfindungsgemäß aufgespalten und das Halogen durch Wasserstoff ersetzt wird. Die Verbindung kann ausschließlich aus Halogen und Kohlenstoff bestehen und somit eine Per-Halogen-Verbindung sein, wie beispielsweise CF₄ oder C₂F₆ oder C₆F₁₄ oder C₈F₁₈. Die Verbindung kann neben Halogen und Kohlenstoff auch Wasserstoff aufweisen, wie beispielsweise CHF₃, CH₂F₂, CF₃CH₃, CHF₂CH₃, etc. Analoge Verbindungen mit Chlor, Brom oder Iod, insbesondere mit Chlor, sind ebenso wie die genannten Verbindungen mit Fluor erfindungsgemäß umfasst. Bevorzugt handelt es sich bei der Verbindung, bei welcher erfindungsgemäß zumindest ein Halogen durch ein Wasserstoff ersetzt wird, um ein Fluoralkan oder ein Chloralkan. Dabei sind perfluorierte Alkane und perchlorierte Alkane von der Bezeichnung mit umfasst.

Erfindungsgemäß kann die Halogenkohlenstoff-Verbindung, welche in dem erfindungsgemäßen Verfahren eingesetzt wird, auch zumindest eine funktionelle Gruppe aufweisen. Bei dieser funktionellen Gruppe kann es sich beispielsweise um eine -COOH oder eine -SO₃H Gruppe handeln. Die zugehörigen Salze sind erfindungsgemäß ebenfalls mit umfasst. Beispielhaft können hier Perfluoroctansulfonsäure oder Perfluoroctancarbonsäure als Verbindung genannt werden.

Überraschenderweise hat sich gezeigt, dass durch eine heterogene Katalyse eine Dehalogenierung halogenierter Kohlenstoffverbindungen unter vergleichsweise milden Bedingungen möglich ist. Da sich der Katalysator nicht verbraucht, ist es ausreichend, wenn geringe Mengen an diesem vorhanden sind, so dass ein wirtschaftliches Verfahren zur Verfügung gestellt werden kann.

Als Metalle, welche als Katalysatoren geeignet sind, sind insbesondere Metalle in gediegenem Zustand zu nennen. Sie können aber auch in Form von Legierungen oder in Form von reinen und gemischten Metallverbindungen oder Oxiden der reinen oder gemischten Metallverbindungen vorliegen.

Als Metalle eignen sich insbesondere die späten Übergangsmetalle, sowie solche, deren Passivierungsschichten oder Oberflächen Lewis-Acide Eigenschaften besitzen. Passivierungsschichten sind beispielsweise oberflächliche Schichten, die durch die Oxidation des Metalls entstanden sind. Als späte Übergangsmetalle sind insbesondere, Platin, Palladium, Nickel, Cobalt, Rhodium, Iridium, Silber und Gold zu nennen. Diese können allein als gediegene Metalle oder als Legierungen, Mischungen der Metalle, Oxide einzelner Metalle oder Mischungen von Metalloxiden verwendet werden. Insbesondere geeignet sind Platin und/oder Palladium.

Die vorliegende Erfindung beschreibt nun erstmals ein Verfahren, in welchem Halogenkohlenstoffverbindungen bzw. Gasströme, die halogenierte Kohlenstoff-verbindungen enthalten, mit einem Reduktionsmittel in Gegenwart von einem Katalysator umgesetzt und dabei dehalogeniert werden. Dabei wird mindestens ein Halogenatom pro Molekül des Halogenkohlenstoffs abgespalten. Als Reduktionsmittel wird Wasserstoff verwendet. Die freie Valenz am Kohlenstoff wird durch ein Wasserstoffatom gesättigt. Das abgespaltene Halogenatom reagiert ebenfalls mit Wasserstoff, so dass als Nebenprodukt Halogenwasserstoffsäure entsteht.

Das Inkontaktbringen zwischen halogenierten Kohlenstoffverbindungen und Wasserstoff am Katalysator erfolgt unter relativ milden Bedingungen, insbesondere wenn man diese mit den Temperaturen aus bisheriger Vernichtung von halogenierten Kohlenstoffverbindungen vergleicht. Erfindungsgemäß erfolgt das Verfahren vorzugsweise bei einer Temperatur von 200 °C bis 800 °C, insbesondere von 400 °C bis 600 °C. Auch Temperaturen von 300 °C oder mehr sind möglich. Temperaturen von 700 °C oder weniger sind besonders bevorzugt. Die genaue Temperatur ist dabei abhängig von der Wahl des Katalysators sowie der halogenierten Kohlenstoffverbindung .

Halogenkohlenstoffverbindungen oder halogenierte Kohlenstoffverbindungen werden im Sinne der vorliegenden Erfindung synonym verwandt.

In einer bevorzugten Ausführungsform handelt es sich bei der Halogenkohlenstoffverbindung um eine Fluorkohlenstoffverbindung und das Verfahren ist ein Verfahren zur Defluorierung derselben.

In einer alternativen, ebenso bevorzugten Ausführungsform ist das vorliegende Verfahren ein Verfahren zur Dechlorierung von Chlorkohlenstoffverbindungen.

Das erfindungsgemäße Verfahren stellt eine heterogene Katalyse dar. Hierfür kann der Katalysator beispielsweise auf einem Träger bereitgestellt werden. Als besonders geeigneter Träger ist Aluminiumoxid anzusehen. Als Katalysator für die Dehalogenierung dient das entsprechende Metall und nicht der Träger.

Das Inkontaktbringen kann beispielsweise in einem Ofen erfolgen, in welchem die Halogen-Kohlenstoffverbindungen und Wasserstoff gemeinsam gasförmig über den Katalysator geleitet werden.

Es ist jedoch erfindungsgemäß auch möglich, dass der Wasserstoff als nasszierender Wasserstoff bereitgestellt wird. Dieser nasszierende Wasserstoff kann auch gasförmig zusammen mit der Halogen-Kohlenstoffverbindung über den Katalysator geleitet werden.

Es ist auch möglich, dass das Inkontaktbringen zwischen der Halogen-Kohlenstoffverbindung und dem Wasserstoff in einem Lösungsmittel erfolgt. Insbesondere in dieser Ausführungsform ist es bevorzugt, wenn der Wasserstoff als nasszierender Wasserstoff bereitgestellt wird, sich also im Lösungsmittel direkt bildet. Es ist jedoch auch in dieser Ausführungsform möglich, dass Wasserstoff gasförmig in das Lösungsmittel eingeleitet wird.

Als Lösungsmittel werden in dieser Ausführungsform insbesondere acide Verbindungen wie Schwefelsäure und andere Mineral- und Carbonsäuren, Alkane, Halogenalkane, Aromatische Lösungsmittel, Alkohole, Ether und/oder Carbonate eingesetzt.

Das erfindungsgemäße Verfahren zeichnet sich zudem dadurch aus, dass Halogen-Kohlenstoffverbindung und Wasserstoff äquimolar zur Verfügung gestellt werden. Nicht umgesetzte Reaktionsströme können erfindungsgemäß erneut als Edukt eingesetzt werden.

Es wird vermutet, dass die erfindungsgemäße Dehalogenierung in einem stufenweisen Prozess erfolgt. So wird vermutet, dass beispielsweise CF₄ zunächst in CHF₃, anschließend zu CH₂F₂, dann weiter zu CH₃F und abschließend zu CH₄, also Methan umgewandelt wird. Eine analoge stufenweise Dehalogenierung erfolgt beispielweise mit CCl₄. Als Produkt wird jeweils Methan erhalten und die jeweilige Halogenwasserstoffsäure, also HF bzw. HCl.

Methan kann als Produkt entweder dem Erdgasnetz oder der thermischen Verwertung zugeführt werden. Alternativ kann Methan als Edukt für chemische Reaktionen benutzt werden.

Als zweites Produkt werden wasserfreie Halogenwasserstoffsäuren, also beispielsweise HF, HCl, HI oder HBr erhalten. Insbesondere wasserfreie Flusssäure (HF, auch a-HF genannt) ist eine hochpreisige Chemikalie, die sich einer erneuten Verwendung zuführen lässt. Entsprechende Produkte sind aus dem bisherigen Verfahren der thermischen Verwertung nicht zu erwarten, so dass das erfindungsgemäße Verfahren auch aus diesem Aspekt wirtschaftlich relevant ist.

In der beigefügten Figur 1 ist ein schematischer Aufbau einer Dehalogenierung am Beispiel einer Defluorierung gezeigt, in welcher Fluorkohlenstoffe aus einem Gasstrom aufbereitet werden. Das Verfahren kann analog auch bei anderen Halogenkohlenstoffverbindungen eingesetzt werden.

Zunächst wird beispielsweise R23, also CHF₃, als Fluorkohlenwasserstoff angenommen, der als Abfallgas vorliegt. Dieses wird mit Wasserstoff gemischt und über einer Platinkatalysator, der auf einen Aluminiumoxidträger aufgebracht ist, bei einer Temperatur von 400 °C bis 600 °C zur Reaktion gebracht. Der austretende Gasstrom kann dahingehend behandelt werden, dass wasserfreie Halogen-Wasserstoffsäure, beispielsweise Flusssäure, verflüssigt und aus dem Gasstrom entfernt wird. Zudem kann Methan aus dem Gasstrom entfernt werden. Dieses kann dann verwertet werden.

Der verbleibende Gasstrom, aus dem die Halogen-Wasserstoffsäure und Methan entfernt wurden, kann erneut der Mischstrecke zugegeben werden und so einer erneuten Reaktion in Gegenwart des Katalysators zugeführt werden.

Es ist somit mit dem erfindungsgemäßen Verfahren möglich, Halogenkohlenwasserstoffe und insbesondere Fluorkohlenwasserstoffe und Chlorkohlenwasserstoffe, zu Methan bzw. Kohlenwasserstoffverbindungen und der entsprechenden Halogenwasserstoffsäure umzuwandeln. Hierbei entstehen keinerlei toxische oder sonst wie umweltschädliche Produkte. Das Verfahren findet zudem bei milden Temperaturen statt, so dass auch der Energiebedarf deutlich geringer als bei bisherigen Verfahren ist.

In den nachfolgenden Ausführungsbeispielen wird die vorliegende Erfindung in nichtemittierender Weise weiter erläutert.

### Ausführungsbeispiele

### Beispiel 1:

CHF₃ wurde mit Wasserstoff in einer entsprechend äquivalenten Menge Wasserstoff in einer Gasphase vermischt. Die Gasmischung wurde über ein temperiertes Katalysatorsystem mit einem Platinkatalysator bzw. einem Palladiumkatalysator geleitet. Diese fanden sich auf Aluminiumoxid als Träger. Das Inkontaktbringen des gemischten Gasstroms, welcher sowohl CHF₃ als auch Wasserstoff enthielt, mit dem Katalysator erfolgte in einem Ofen bei einer Temperatur von 400 °C bis 600 °C. Das Gas wurde kontinuierlich über den Katalysator geleitet.

Der erhaltene Gasstrom wurde mittels Gaschromatografie analysiert. Es wurden als Produkte CHF₃, CH₂F₂ und CH₃F erhalten. Als Hauptprodukt enthielt der Gasstrom CH₄. Daneben konnte wasserfreies HF identifiziert werden.

### Beispiel 2:

Analog wie in Beispiel 1 wurde CHF₃ gemeinsam mit Wasserstoff über einen Katalysator geleitet. Als Katalysator wurde Nickel eingesetzt.

Der erhaltene Gasstrom wurde mittels Gaschromatografie analysiert. Es wurden als Produkte CHF₃, CH₂F₂ und CH₃F erhalten. Als Hauptprodukt enthielt der Gasstrom CH₄.

### Beispiel 3:

Analog wie in Beispiel 1 wurde CH₂F₂ gemeinsam mit Wasserstoff über einen Katalysator geleitet. Als Katalysator wurde Platin oder Palladium auf Aluminiumoxid verwendet.

Der erhaltene Gasstrom wurde mittels Gaschromatografie analysiert. Es wurden als Produkte CH₂F₂ und CH₃F erhalten. Als Hauptprodukt enthielt der Gasstrom CH₄.

### Beispiel 4:

Analog wie in Beispiel 1 wurde CH₃F gemeinsam mit Wasserstoff über einen Katalysator geleitet. Als Katalysator wurde Platin oder Palladium auf Aluminiumoxid verwendet.

Der erhaltene Gasstrom wurde mittels Gaschromatografie analysiert. Es wurden als Produkte CH₂F₂ und CH₃F erhalten. Als Hauptprodukt enthielt der Gasstrom CH₄.

### Beispiel 5:

Analog wie in Beispiel 1 wurde CHF₃ gemeinsam mit Deuterium über einen Katalysator geleitet. Als Katalysator wurde Platin oder Palladium auf Aluminiumoxid verwendet.

Der erhaltene Gasstrom wurde mittels Gaschromatografie analysiert. Es wurden als Produkte CHDF₂ und CHD₂F erhalten. Als Hauptprodukt enthielt der Gasstrom CHD3.

## Patentansprüche

1. Verfahren zur Dehalogenierung von Halogenkohlenstoff-Verbindungen, **dadurch gekennzeichnet, dass** man die Halogenkohlenstoff-Verbindung in Gegenwart eines Katalysators mit elementarem Wasserstoff in Kontakt bringt, wobei der Katalysator ein Metallkatalysator ist.

2. Verfahren nach Anspruch 1, **dadurch gekennzeichnet, dass** als Metallkatalysator Metalle in gediegenem Zustand, in Form von Legierungen und in Form von reinen und gemischten Metallverbindungen und Oxiden verwendet werden, wobei das Metall/die Metalle ausgewählt sind aus Pt, Pd, Ni, Co, Rh, Ir, Ag und/oder Au und solche, deren Oberflächen und oder Passivierungsschichten Lewis-Acide Eigenschaften besitzen.

3. Verfahren nach Anspruch 2, **dadurch gekennzeichnet, dass** der Katalysator ausgewählt ist aus Pt, Pd, Ni, Ag, Co, Rh, Ir und/oder Au, insbesondere aus Pt und/oder Pd und/oder Ni.

4. Verfahren nach zumindest einem der Ansprüche 1 bis 3, **dadurch gekennzeichnet, dass** das in Kontakt bringen bei einer Temperatur im Bereich von 200 °C bis 800 °C, insbesondere von 400 °C bis 600 °C erfolgt.

5. Verfahren nach zumindest einem der Ansprüche 1 bis 4, **dadurch gekennzeichnet, dass** der Katalysator auf einen Träger, insbesondere Aluminiumoxid, aufgebracht wird.

6. Verfahren nach zumindest einem der Ansprüche 1 bis 5, **dadurch gekennzeichnet, dass** es ein Verfahren zur Defluorierung von Fluorkohlenstoffverbindungen ist.

7. Verfahren nach zumindest einem der Ansprüche 1 bis 5, **dadurch gekennzeichnet, dass** es ein Verfahren zur Dechlorierung von Chlorkohlenstoffverbindungen ist.

8. Verfahren nach zumindest einem der Ansprüche 1 bis 7, **dadurch gekennzeichnet, dass** Halogenkohlenstoff-Verbindung und Wasserstoff gasförmig über den Katalysator geleitet werden.

9. Verfahren nach zumindest einem der Ansprüche 1 bis 8, **dadurch gekennzeichnet, dass** der Wasserstoff als nascierender Wasserstoff bereitgestellt wird.

10. Verfahren nach zumindest einem der Ansprüche 1 bis 9, **dadurch gekennzeichnet, dass** das in Kontakt bringen in einem Lösungsmittel erfolgt.

11. Verfahren nach Anspruch 10, **dadurch gekennzeichnet, dass** das Lösungsmittel ausgewählt ist aus Schwefelsäure und anderen Mineral- und Carbonsäuren, Alkanen, Halogenalkanen, Aromatischen Lösungsmitteln, Alkoholen, Ethern und/oder Carbonaten.
